# EUROPEAN PATENT APPLICATION

(11) **EP 2 674 414 A1**
(43) Date of publication of application: **18.12.2013**
(21) Application number: 12004544.8
(22) Date of filing: 15.06.2012
(51) Int. Cl.: C07C 215/54, C07C 217/62, C07C 235/34

(54) **Method for the preparation of 1-aryl-1-alkyl-3-dialkylaminopropane compounds**

(71) Applicant: Siegfried AG, 4800 Zofingen (CH)
(72) Inventor: Cyr, Patrick, Wilmington DE 19808 (US); Crump, Roger, Woolwich TWP NJ 08085 (US); Sahli, Stefan, 4800 Zofingen (CH); Blum, Vinzenz, 6243 Egolzwil (CH)
(74) Representative: Schaad, Balass, Menzl & Partner AG

(57) **Abstract**

The present invention refers to the preparation of 1-aryl-1-alkyl-2-alkyl-3-dialkylamino-propane compounds, such as tapentadol, using a diastereoselective Eschenmoser-Claisen or Ireland-Claisen rearrangement.

## Description

The present invention refers to a novel method for the preparation of 1-aryl-1-alkyl-2-alkyl-3-dialkylamino-propane compounds involving an Eschenmoser-Claisen or an Ireland-Claisen rearrangement.

Among the known 1-aryl-1-alkyl-2-alkyl-3-dialkylamino-propane compounds, 3-[(1*R*,2*R*)-3-(dimethylamino)-1-ethyl-2-methyl-propyl]phenol (tapentadol) is probably the most famous. Tapentadol (formula IXa; see below) is a centrally acting analgesic with a dual mode of action as an agonist at the µ-opioid receptor and as a norepinephrine reuptake inhibitor. It is generally used for the treatment of moderate to grave acute pain.

To date, several methods for the preparation of tapentadol and other 1-aryl-1-alkyl-2-alkyl-3-dialkylamino-propane compounds are known:
USRE 39493 discloses the substance tapentadol and other 3-phenyl-1-dimethylaminopropane compounds, while US 2002/0010178 from the same patent family discloses methods for the preparation of tapentadol and its derivatives. These methods focus on elimination or substitution reactions of halogen or hydroxyl groups at the 3-position of the propane moiety.

US 5,811,582 describes dimethyl-(3-aryl-but-3-enyl)amine compounds as pharmaceutical active ingredients, following a very similar synthetic route as that of US 2002/0010178. US 2006/0194988 and US 2006/0167318 both describe methods involving the dehydration of substituted 1-amino-3-aryl-butan-3-ol compounds, followed by a hydrogenation of the double bond.

US 2009/0043132 refers to a method for the preparation of O-methyl tapentadol and related compounds, featuring the hydrogenation of substituted dimethyl-(3-aryl-but-3-enyl) amine compounds.

US 2010/0099916 also describes a synthesis of tapentadol using a Grignard addition reaction, followed by dehydration and hydrogenation.

US 2009/0326271 and US 2009/0312578 both disclose the acylation of substituted 1-amino-3-aryl-butan-3-ol compounds to obtain the corresponding triflate, followed by diastereoselective elimination and hydrogenation of the resulting C=C double bond.

WO 2011/067714 describes intermediates with nitro substituted phenols in the synthesis of tapentadol.

WO 2011/080756 discloses processes for the preparation of 1-phenyl-3-dimethylaminopropane derivatives starting from a cyano intermediate. The cyano intermediate is (i) reduced to an amine, followed by methylation, or (ii) hydrolyzed to an acid, followed by conversion to an amide and reduction.

WO 2011/080736 and US 2011/0306793 refer to methods for the preparation of tapentadol and related compounds starting from 1-amid-3-aryl-prop-2-en compounds.

WO 2011/092719 describes processes starting from 1-hydroxy-3-aryl-propan compounds.

WO 2011/128784 discloses the synthesis of tapentadol via 1-aryl-1-hydroxy-3-methylphenylaminopropan compounds.

WO 2012/001571 describes processes via 1-aryl-1-hydroxy-3-dimethylaminopropan compounds.

However, the above methods lack either economic efficiency or practical application, or they are expensive and/or involve the use of toxic materials. Therefore, there is still a need for an improved method for the preparation of tapentadol, in particular on large scale.

This problem is solved by the method according to claim 1, the use according to claim 12, and the intermediates according to claims 13, 14, and 15. Preferred embodiments are subject of the dependent claims.

The present invention refers to a new method for the preparation of a 1-aryl-1-alkyl-2-alkyl-3-dialkylamino-propane compound having the general formula (I)

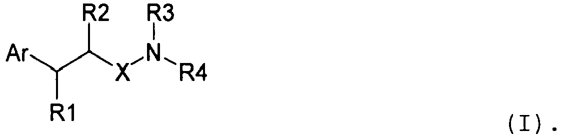

In formula (I) and throughout this application, the substituents are defined as follows:
- Ar is a substituted or unsubstituted aryl group, such as phenyl, pyridyl or paphthyl, and in particular a substituted phenyl group. The aryl group may be mono- or multi-substituted, bearing substituents in ortho-, meta- and/or para-position. Preferably, the aromatic group is a phenyl group, which bears one additional substituent in meta-position, i.e. in 3-position. The optional substituent on the phenyl group may be, for instance, a protected or unprotected hydroxyl group, a protected amino group, a halogen or a nitro group.
- R1 is a linear, branched or cyclic C₂ to C₄ alkyl or alkenyl group.
- R2 is a linear, branched or cyclic C₁ to C₄ alkyl group.
- R3 and R4 each are independently of one another a linear, branched or cyclic C₁ to C₄ alkyl group, hydrogen or an optionally substituted benzyl group or R3 and R4 together form a cyclopentyl, cyclohexyl, tetrahydrofuryl or tetrahydropyryl group.
- X is a methylene (i.e. -CH₂-) or a carbonyl (i.e. C=O) group.

The method of the present invention is **characterized in that** it involves an Eschenmoser-Claisen or an Ireland-Claisen rearrangement. Preferably, the method involves an Eschenmoser-Claisen rearrangement.

Eschenmoser-Claisen and Ireland-Claisen rearrangements are well known in chemical synthesis.

In an Eschenmoser-Claisen rearrangement, an allylic alcohol is converted to a γ,δ-unsaturated amide in a stereospecific fashion (R, R', and R" being arbitrary substituents in this particular reaction scheme): 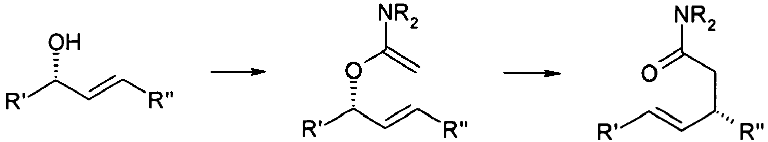 Eschenmoser-Claisen rearrangements are generally carried out in aromatic solvents, such as benzene, toluene or xylene, under reflux.

In an Ireland-Claisen rearrangement, an allylic alcohol is esterified and then converted to a γ,δ-unsaturated carboxylic acid in a stereospecific fashion (R, R', and R" being arbitrary substituents in this particular reaction scheme):

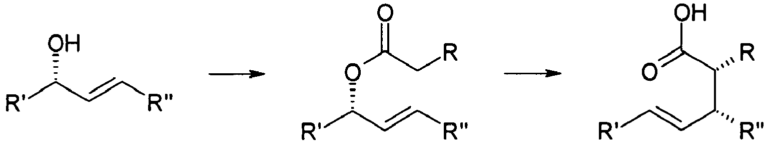

Ireland-Claisen rearrangements are generally carried out using a non-alkylating base, such as triethylamine or *N,N-*ethyldiisopropyl amine, in an organic solvent, such as hexanes, toluene, benzene, xylene or methylene chloride, at low temperature.

In the method of the present invention, the Eschenmoser-Claisen or Ireland-Claisen rearrangement allows for a very efficient and straight forward synthesis of the 1-aryl-1-alkyl-2-alkyl-3-dialkylamino-propane compounds of formula (I). In particular, the synthesis is less complex than previous ones, and also requires less toxic materials.

Furthermore, the Eschenmoser-Claisen and Ireland-Claisen rearrangement, respectively, proceeds in a highly diastereoselective fashion and affords the desired product in high yield. This makes the method of the present invention also economically very efficient.

The method of the present invention is particularly well suited for the preparation of tapentadol, its precursors, and related compounds.

To this end, it is particularly preferred if the 1-aryl-1-alkyl-2-alkyl-3-dialkylamino-propane compounds formed in the method of the present invention have the same relative and/or absolute stereochemistry as tapentadol.

Therefore, according to a preferred embodiment, a compound of formula (Ia) 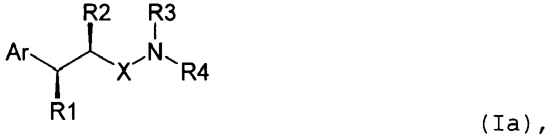 the other enantiomer of (Ia), or a mixture of the two enantiomers is formed by the method of the present invention. These compounds have the same relative stereochemistry as tapentadol, with the depicted enantiomer (Ia) having also the same absolute stereochemistry as tapentadol. Therefore, they are very well suited as advanced intermediates in a synthesis of tapentadol. Furthermore, the other functional groups present in these compounds allow for an easy conversion to tapentadol. As will be well appreciated by the person skilled in the art, the above compounds may also be used for the preparation of other compounds having a similar structure as tapentadol, of course.

Throughout this application, a "mixture of two enantiomers" always refers to all possible proportions of the two enantiomers, including a racemic mixture.

According to a preferred embodiment, the method of the present invention involves reacting a compound of formula (II) 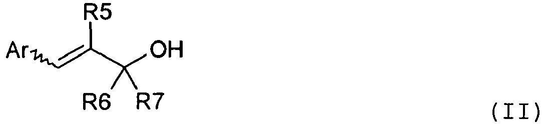 with a compound of formula (III) or (IV) 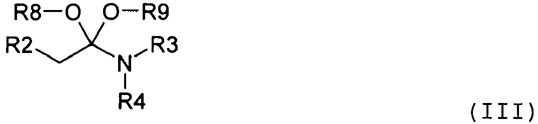 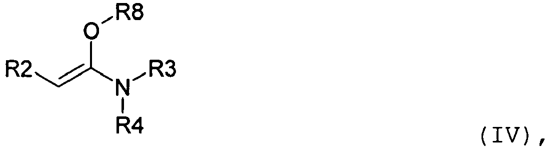 to afford a compound of formula (V)

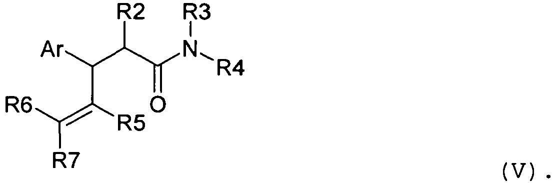

In the above formulas, Ar, R2, R3, and R4 are as defined above, R5, R6, and R7 are independently of one another selected from the group consisting of hydrogen and methyl, and R8 and R9 are independently of one another a linear, branched or cyclic C₁ to C₄ alkyl group or an optionally substituted benzyl group. Preferably, R6 and R7 are hydrogen.

This reaction corresponds to an Eschenmoser-Claisen rearrangement.

More preferably, the method of the present invention involves reacting a compound of formula (II) with a compound of formula (III) or (IV) to afford a compound of formula (Va) 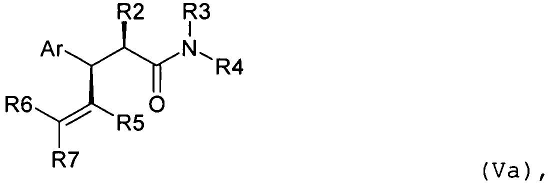 to afford the other enantiomer of (Va) or to afford a mixture of the two enantiomers.

In the above reaction, the allylic alcohol (II) will react with (III) or (IV) under suitable reaction conditions to afford an intermediate of the general formula (X)

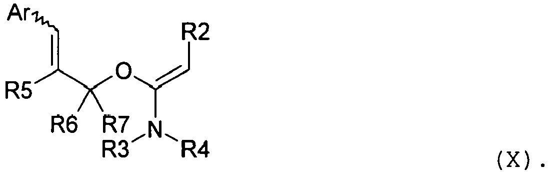

The intermediate (X) may be formed in a separate reaction step and isolated, or it may be formed *in situ* and processed further without isolation.

Upon heat treatment, intermediate (X) undergoes a [3,3]-sigmatropic rearrangement to afford (V). This rearrangement is generally stereospecific and high yielding. Thus, by choosing the E- or Z-allylic alcohol (II), the stereochemistry of the product (V) can be determined.

Preferably, the *E*-allylic alcohol (II) is used, as this will lead to the formation of the syn-product (as in (Va), for instance).

Within the method of the present invention, the amide compounds (V) thus obtained may then be further converted to more advanced intermediates and ultimately to tapentadol. Alternatively, it is also possible to prepare other compounds, which are structurally closely related to tapentadol.

According to a preferred embodiment, the aryl group Ar is selected from the group consisting of 3-hydroxyphenyl, 3-methoxyphenyl, 3-benzyloxyphenyl, and O-protected 3-hydroxyphenyl. Alternatively, the second substituent may also be arranged in 2- or 4-position of the aryl group. It has been found that both the Eschenmoser-Claisen and the Ireland-Claisen rearrangement work well for these aromatic groups. Furthermore, these aromatic groups can easily be converted to a 3-hydroxy-phenyl group, i.e. the aromatic group of tapentadol. As will be well understood by the person skilled in the art, similar aromatic groups are also present in other compounds related to tapentadol and in their derivatives, and may therefore be freely combined with the other substituents disclosed herein.

According to a preferred embodiment, R1 is selected from the group consisting of ethyl, ethenyl, propyl, propenyl, iso-propyl, *iso*-propenyl, butyl, butenyl, *iso*-butyl, and *iso*-butenyl. In tapentadol and its precursors, R1 is an ethyl or ethenyl group. For this reason, it is particularly preferred that R1 is an ethyl or an ethenyl group. However, by choosing a different R1 group, it is possible to prepare an analogue of tapentadol.

According to a preferred embodiment, R2 is selected from the group consisting of methyl, ethyl, propyl, iso-propyl, butyl, and *iso*-butyl. In tapentadol and its precursors, R2 is a methyl group. For this reason, it is particularly preferred that R2 is a methyl group. However, by choosing a different R2 group, it is possible to prepare an analogue of tapentadol.

According to a preferred embodiment, R3 and R4 are independently of one another selected from the group consisting of methyl, ethyl, propyl, *iso*-propyl, butyl, *iso*-butyl, and optionally substituted benzyl or R3 and R4 together form a cyclopentyl, cyclohexyl, tetrahydrofuryl or tetrahydropyryl group. In tapentadol and its precursors, R3 and R4 are both methyl groups. For this reason, it is particularly preferred that R3 and R4 are methyl groups. However, by choosing different R3 and R4 groups, it is possible to prepare an analogue of tapentadol. Alternatively, if R3 and/or R4 are substituted or unsubstituted benzyl groups, they may easily be converted to methyl groups by hydrogenation followed by reductive alkylation or alkylation with an electrophile.

According to a preferred embodiment, a compound of formula (VI) 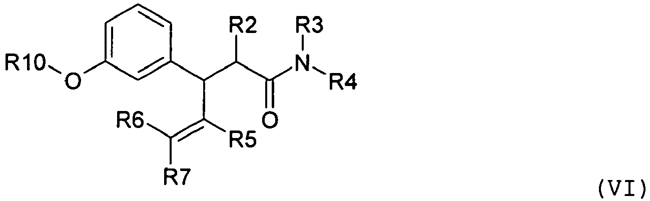 is formed by the method of the present invention.

More preferably, a compound of formula (VIa) 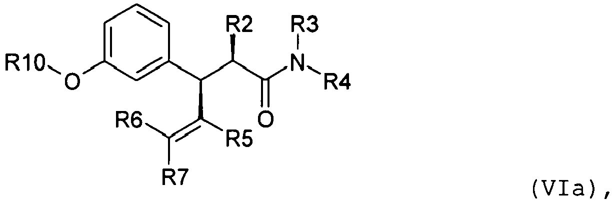 the other enantiomer of (VIa) or a mixture of the two enantiomers is formed.

In the above structures (VI) and (VIa), R2, R3, R4, R5, R6, and R7 are as previously defined and R10 is selected from the group consisting of hydrogen, methyl, benzyl, and an alcohol protecting group. Suitable alcohol protecting groups include alkoxycarbonyl groups, such as benzyloxycarbonyl, *p*-methoxybenzyl, methyloxymethyl, benzyloxymethyl, methylthiomethyl, tetrahydropyranyl, allyl, acetyl, *p*-touylsulfonyl or silyl groups, and in particular silyl groups, such as *tert*-butyldimethyl silyl.

More preferably, R2, R3, R4, and R10 are methyl groups and R5, R6, and R7 are hydrogen.

The above compounds are particularly well suited for the synthesis of tapentadol. Furthermore, their preparation is easily achieved by the method of the present invention involving an Eschenmoser-Claisen or an Ireland-Claisen rearrangement. In the latter case, the carboxylic acid obtained in the rearrangement is easily converted to the corresponding amide using thionyl chloride or oxalyl chloride, followed by treamtment with dimethylamine. Thanks to the stereospecificity of the rearrangement, the compounds of formula (VI) can be prepared in a highly diastereoselective fashion.

According to a preferred embodiment, a compound of formula (VII) 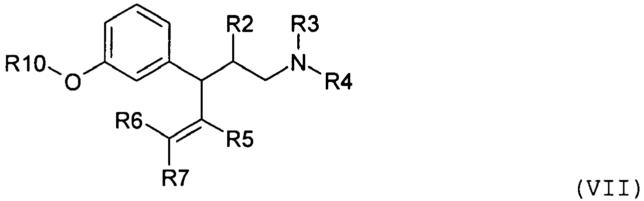 is formed by the method of the present invention.

More preferably, a compound of formula (VIIa) 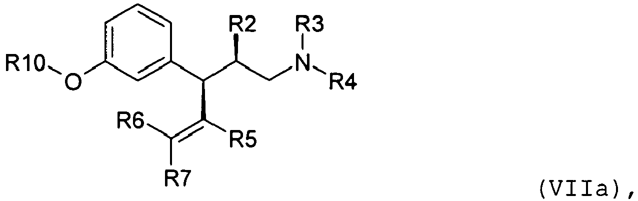 the other enantiomer of (VIIa) or a mixture of the two enantiomers is formed.

In the above structures (VII) and (VIIa), R2, R3, R4, R5, R6, R7, and R10 are as previously defined.

More preferably, R2, R3, R4, and R10 are methyl groups and R5, R6, and R7 are hydrogen.

The above compounds are particularly well suited for the synthesis of tapentadol. Furthermore, their preparation is easily achieved by the method of the present invention involving an Eschenmoser-Claisen or Ireland-Claisen rearrangement. For instance, the amines (VII) can be obtained by reduction of the corresponding amide (VI) under standard conditions. Furthermore, thanks to the stereospecificity of the Eschenmoser-Claisen or Ireland-Claisen rearrangement, the compounds of formula (VII) can be prepared in a highly diastereoselective fashion.

Also, an enantiomeric mixture of such an amine (VIIa) is very well suited for a separation of the two enantiomers, for instance by treatment with an enantiomerically pure chiral acid, such as tartaric acid and its derivatives. Preferably, the separation is brought about by kinetic resolution.

Alternatively, it would also be possible to use a chiral catalyst for the Eschenmoser-Claisen or Ireland-Claisen rearrangement, such that the desired enantiomer is obtained directly from the rearrangement. In this manner, an additional separation step can be avoided.

According to a preferred embodiment, a compound of formula (VIII) 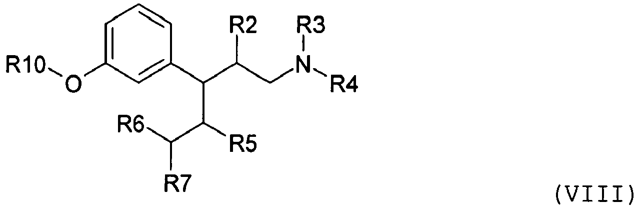 is formed by the method of the present invention.

More preferably, a compound of formula (VIIIa) 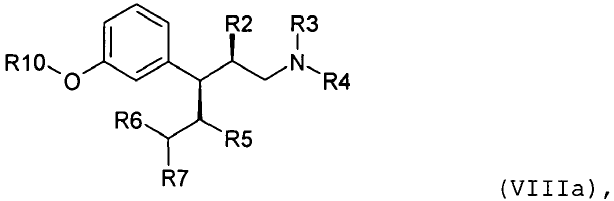 the other enantiomer of (VIIIa) or a mixture of the two enantiomers is formed.

In the above structures (VIII) and (VIIIa), R2, R3, R4, R5, R6, R7, and R10 are as previously defined.

More preferably, R2, R3, R4, and R10 are methyl groups and R5, R6, and R7 are hydrogen.

The above compounds are particularly well suited for the synthesis of tapentadol. Furthermore, their preparation is easily achieved by the method of the present invention involving an Eschenmoser-Claisen or Ireland-Claisen rearrangement. For instance, the compounds of formula (VIII) can be obtained by hydrogenation of the corresponding alkene (VII) under standard conditions. Furthermore, thanks to the stereospecificity of the Eschenmoser-Claisen or Ireland-Claisen rearrangement, the compounds of formula (VIII) can be prepared in a highly diastereoselective fashion.

According to a preferred embodiment of the method of the present invention, the compound of formula (IX) 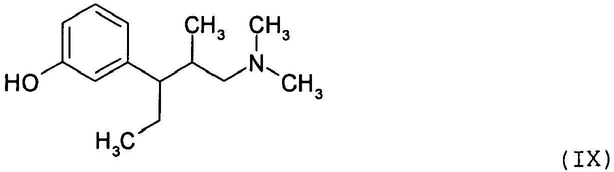 is formed.

More preferably, a compound of formula (IXa) 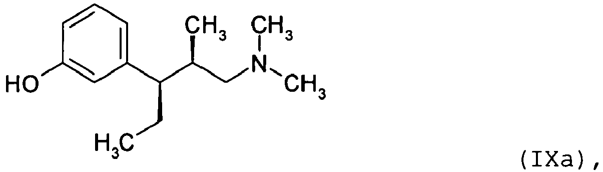 the other enantiomer of (IXa) or a mixture of the two enantiomers is formed in the method of the present invention.

Most preferably, only (IXa), i.e. tapentadol, is formed.

The synthesis of the above compounds, and in particular of tapentadol (IXa), can be achieved by the following reactions steps, for instance, involving an Eschenmoser-Claisen rearrangement (Scheme 1):

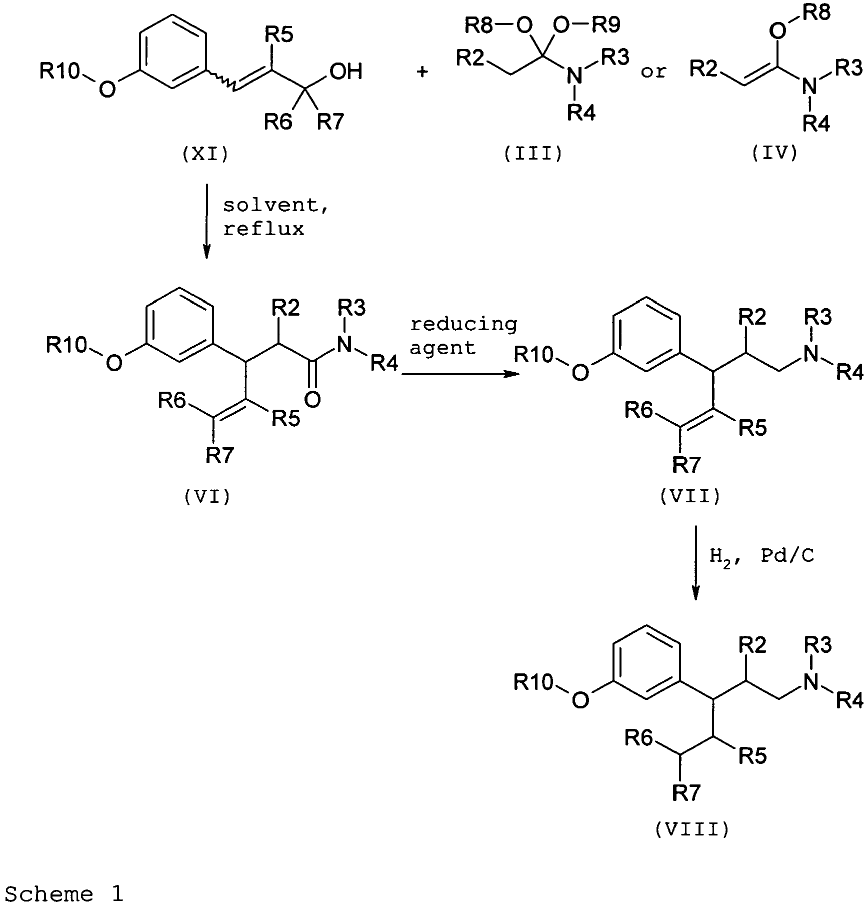

Alternatively, the synthesis of the above compounds, and in particular of tapentadol (IXa), can be achieved by the following reactions steps, for instance, involving an Ireland-Claisen rearrangement (Scheme 2):

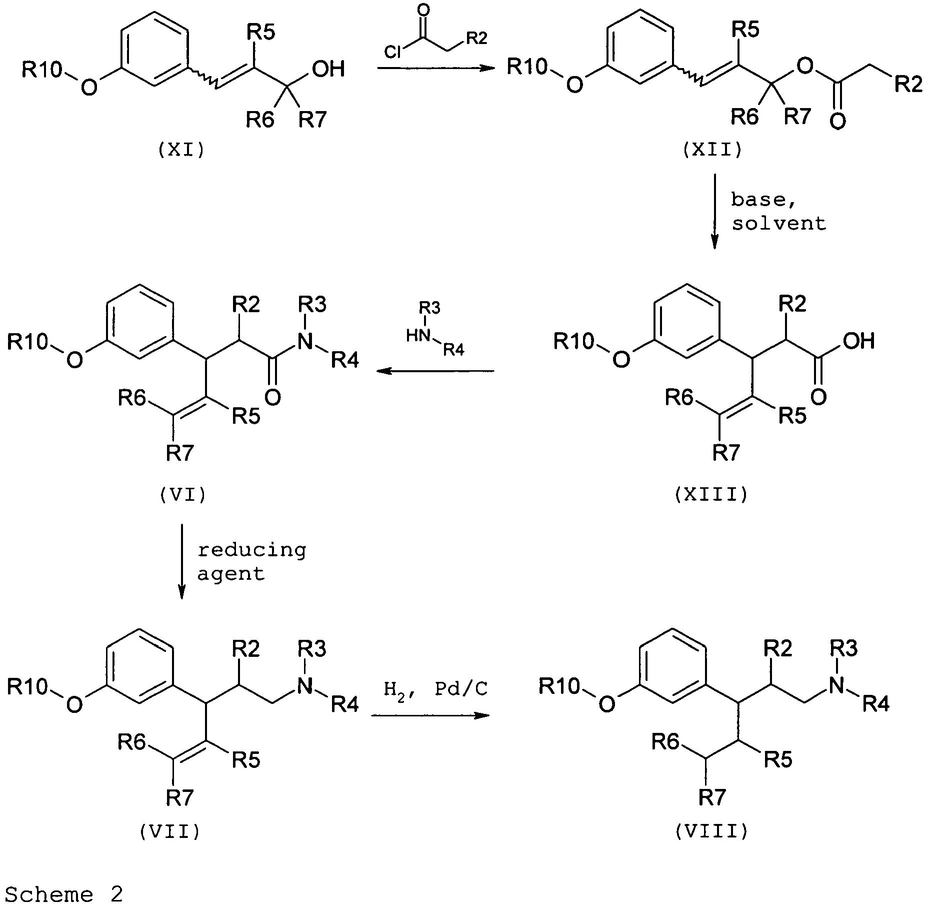

Racemic (IX) can be obtained if R2, R3, and R4, are methyl and R5, R6, and R7 are hydrogen. In order to obtain tapentadol (IXa) as a single enantiomer, a kinetic resolution, e.g. of (VIII), may be employed. Furthermore, the phenolic alcohol may be protected by a protecting group R8, such as methyl, which is ultimately removed in order to obtain tapentadol.

As has been shown above, the method of the present invention is ideal for the synthesis of tapentadol. This is in particular due to the highly stereoselective Eschenmoser-Claisen or Ireland-Claisen rearrangement employed.

Therefore, in a further aspect, the present invention also refers to the use of an Eschenmoser-Claisen or an Ireland-Claisen rearrangement for the preparation of tapentadol (IXa). It has been found that for the compounds used in the present invention, both the Eschenmoser-Claisen and the Ireland-Claisen rearrangement proceeds in a highly diastereoselective fashion. Therefore, if the reaction is carried out on the *E*-allylic alcohol, the 2,3-*syn*-amide (Va) or -ester is obtained. The Z-allylic alcohol, on the other hand, leads to the formation of the 2,3-anti-amide or -ester (not shown).

In the course of the method of the present invention, several important intermediates are formed, which are all closely related to each other. Among these intermediates, the most important ones are: 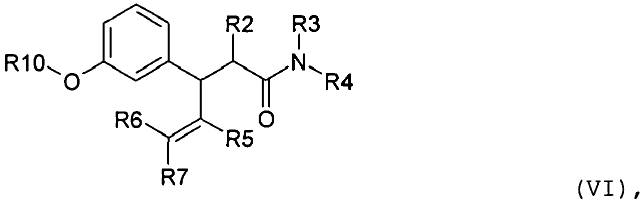 more preferably 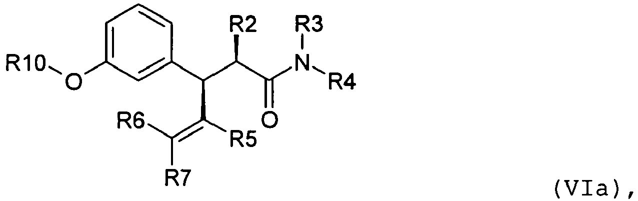 the other enantiomer of (VIa) or a mixture of the two enantiomers; 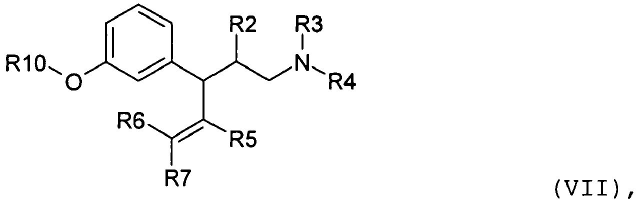 more preferably 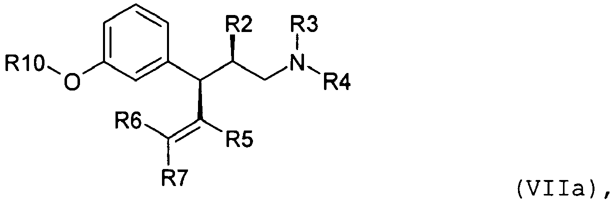 the other enantiomer of (VIIa) or a mixture of the two enantiomers; and 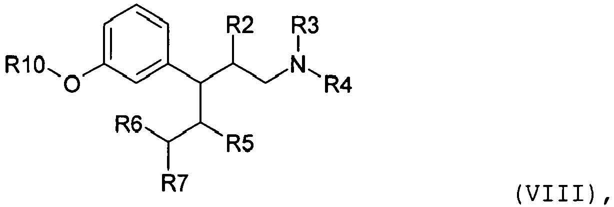 more preferably 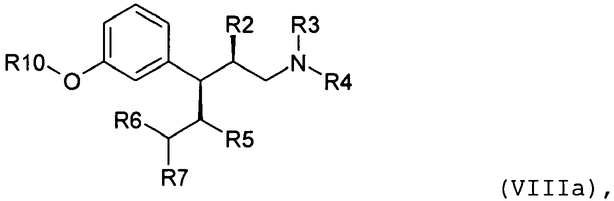 the other enantiomer or a mixture of the two enantiomers.

In the above intermediates, R2 is a linear, branched or cyclic C₁ to C₄ alkyl group; R3 and R4 each are independently of one another a linear, branched or cyclic C₁ to C₄ alkyl group, hydrogen or an optionally substituted benzyl group or R3 and R4 together form a cyclopentyl, cyclohexyl, tetrahydrofuryl or tetrahydropyryl group; R5, R6, and R7 are independently of one another selected from the group consisting of hydrogen and methyl; and R10 is selected from the group consisting of hydrogen, methyl, benzyl, and an alcohol protecting group. More preferably, R2, R3, R4, and R10 are methyl and R5, R6, and R7 are hydrogen.

The most preferred intermediates include, but are not limited to:

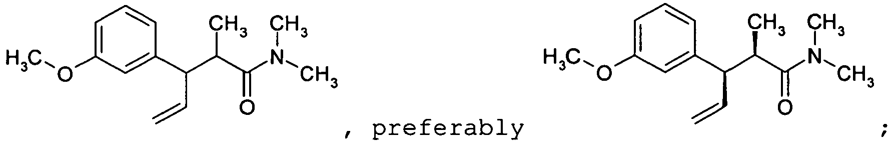

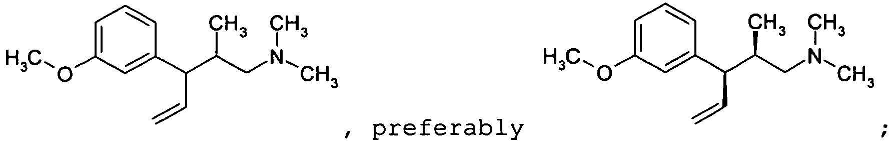

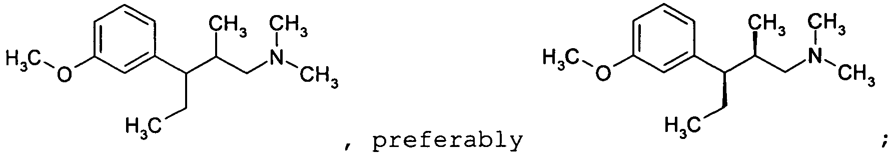

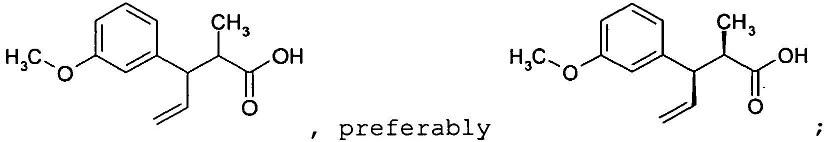

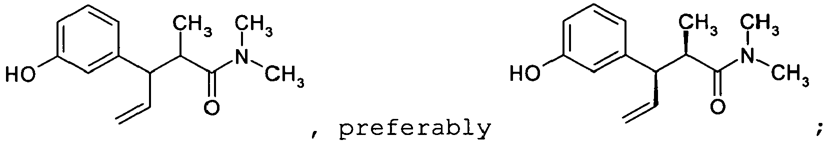

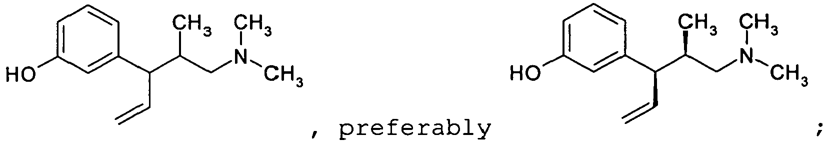

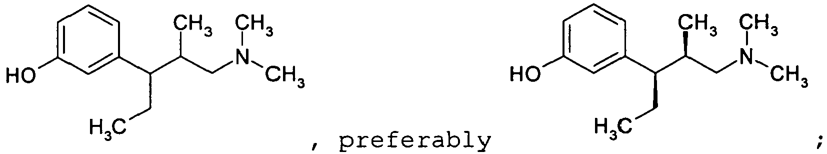

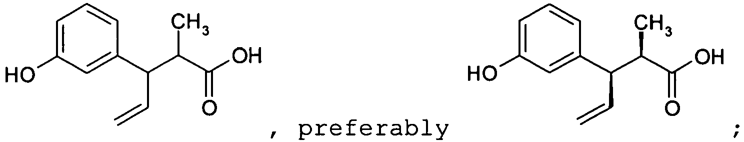

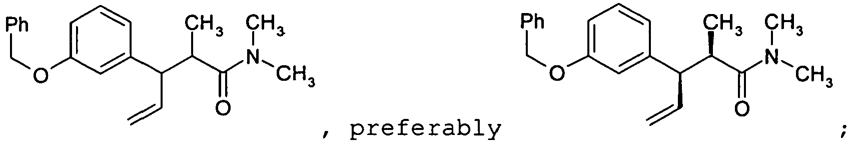

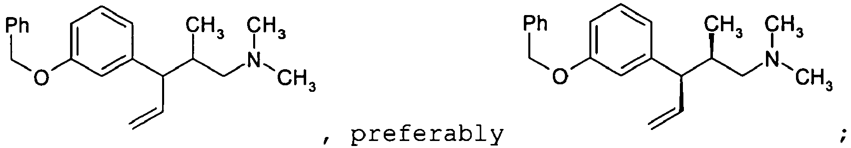

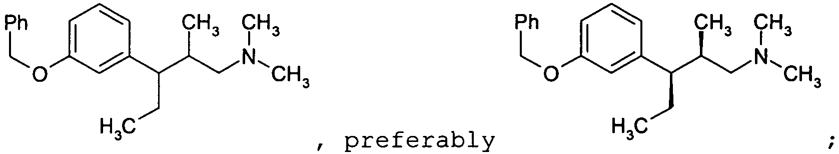

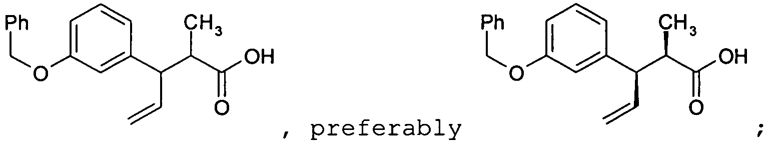

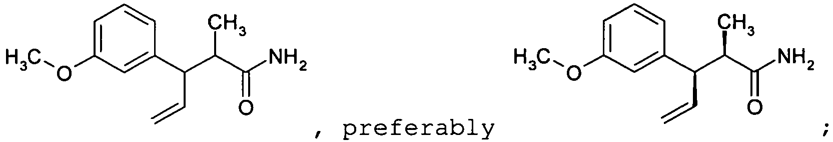

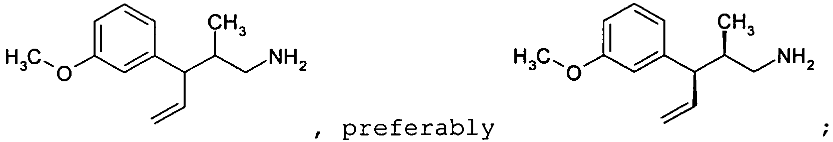

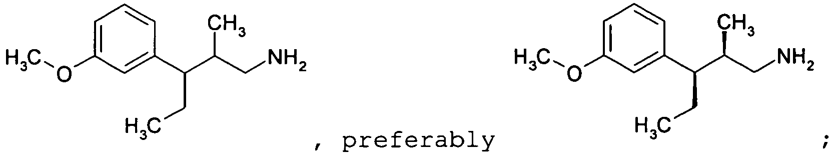

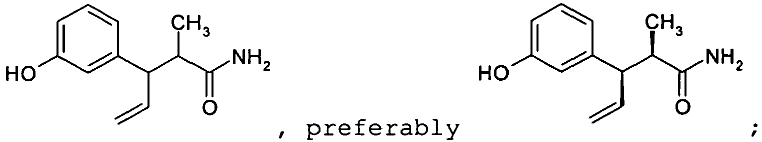

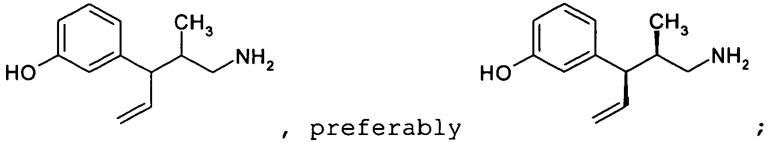

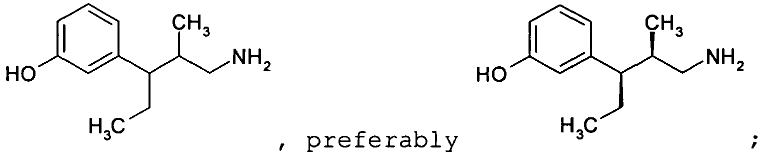

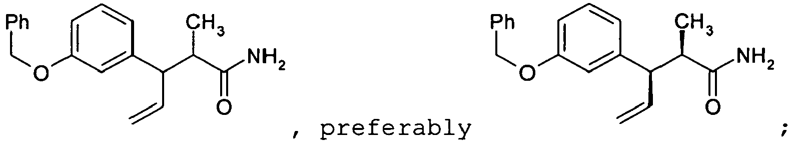

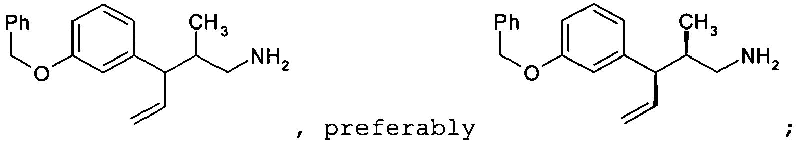

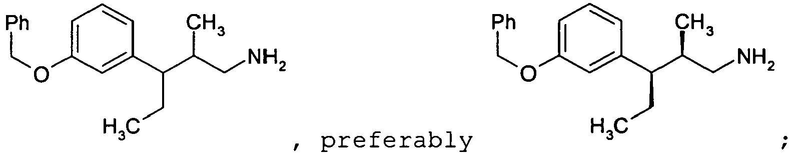

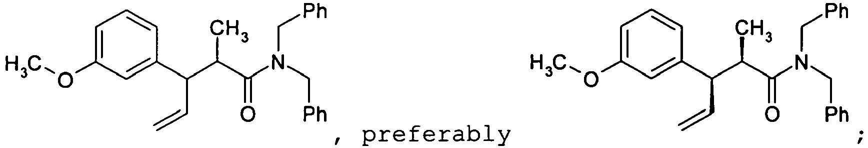

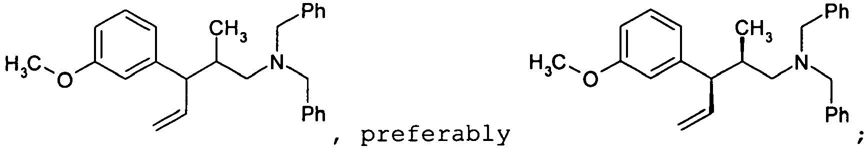

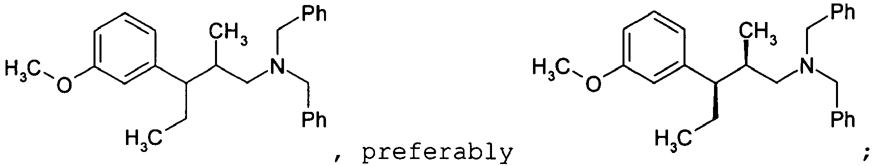

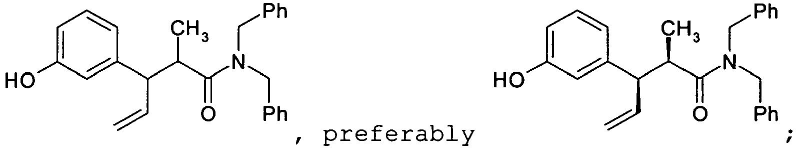

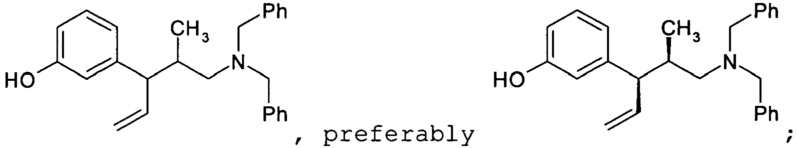

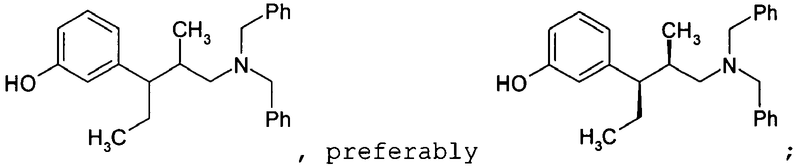

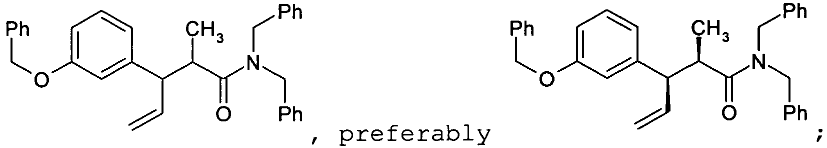

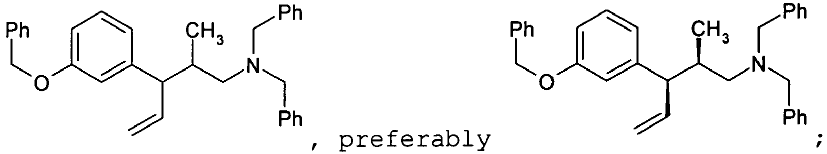

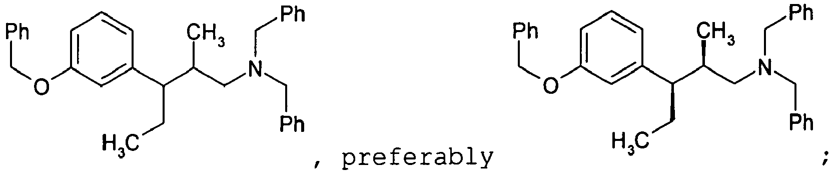

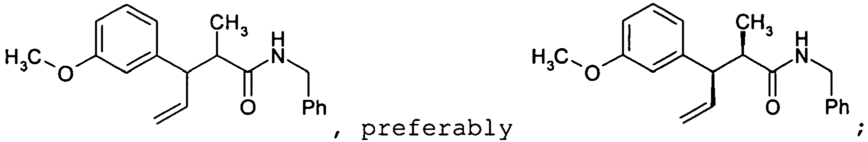

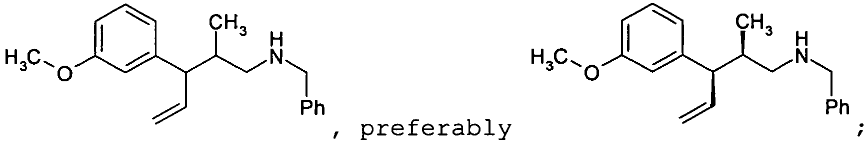

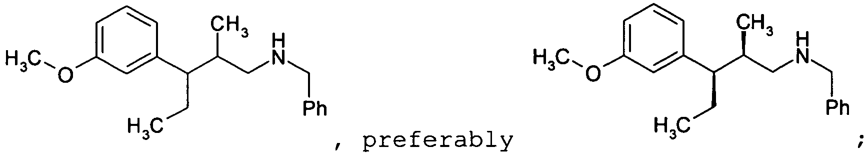

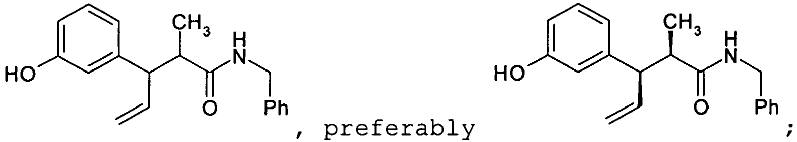

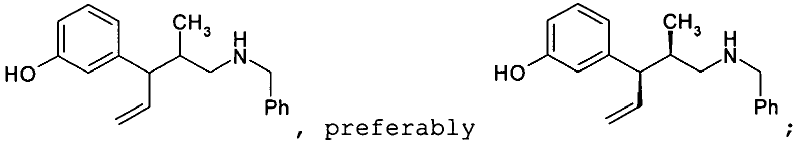

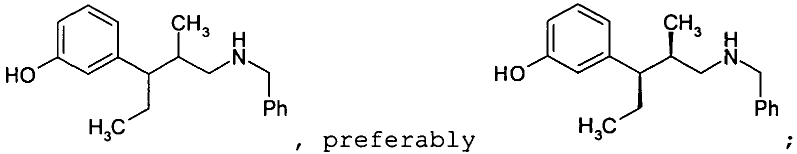

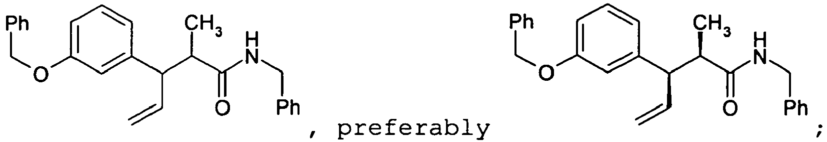

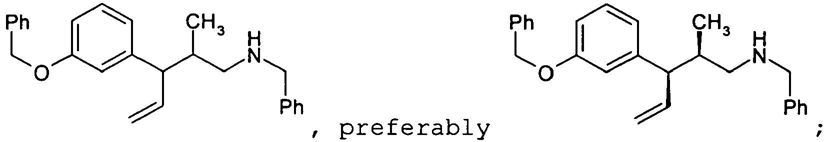

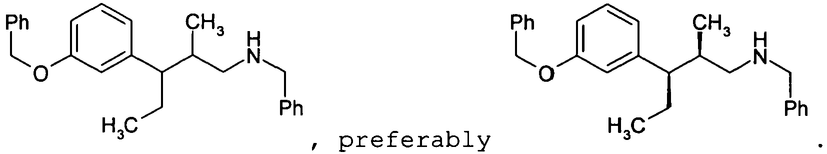

The present invention will be further illustrated by way of the following examples:

### Example 1: Preparation of (E)-3-(3-Methoxyphenyl)-prop-2-en-1-ol (XV)

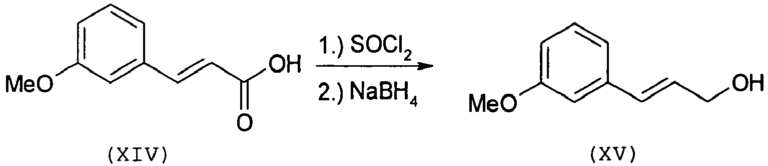

3-Methoxycinnamic acid (XIV; 14.6 g, 82 mmol) was mixed with CH₂Cl₂ (200 g) and treated with SOCl₂ (37.7 g, 317 mmol) at 22 °C. The mixture was stirred for 3 h at 22 °C and then concentrated in vacuo. Additional CH₂Cl₂ (50 g) was added to the residue and the resulting mixture concentrated in vacuo. 16 g of a brownish oil were obtained that started to crystallize (acyl chloride). 40 g of acetonitrile were added to the above brownish oil. The resulting solution was treated with a solution of NaBH₄ (3.75 g, 99 mmol), Na₂CO₃ (10 g, 94 mmol), and water (80 g) at 5 - 13 °C. The mixture was stirred for 30 minutes at 10 °C and then warmed to 22 °C. Aq. NaOH-solution (10%, 50 g) was added at 22 °C, and the mixture stirred for 15 minutes and then extracted with Et₂O (72 g). The organic phase was washed with brine (50 g), dried over MgSO₄, filtered and concentrated in vacuo. 14 g of yellow oil were obtained as crude product.

Purification by column chromatography (SiO₂; CH₂Cl₂:MeOH 97:3) afforded 9.9 g of *(E)*-3-(3-Methoxyphenyl)-prop-2-en-1-ol (XV) as a yellowish oil, corresponding to 74% yield.

### Example 2: Preparation of Trimethylpropionic imidinium methylsulfate (XVII)

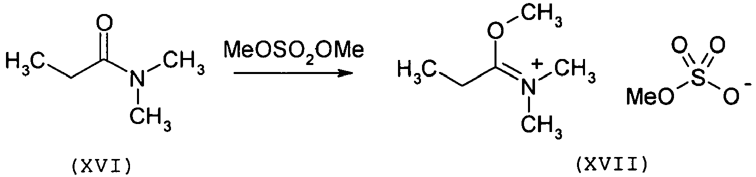

A mixture of N,N-dimethylpropionic amide (XVI; 25 g, 247 mmol) and dimethyl sulfate (30 g, 238 mmol) were heated to 82 °C and stirred for 2.5 h. After cooling to 22 °C, toluene (55 g) was added and stirred for 20 minutes before separating the phases.

The upper toluene phase was disposed and the lower product phase extracted with Et₂O (45 g) for 10 minutes. After the phase separation, the lower product phase was concentrated in vacuo to afford 39.8 g of trimethylpropionic imidinium methylsulfate (XVII) as a yellow oil in 71% yield, which was stored over 3 Å molecular sieves.

### Example 3: Preparation of 1,1-Dimethoxy-N,N-dimethyl-propan-1-amine (XVIII)

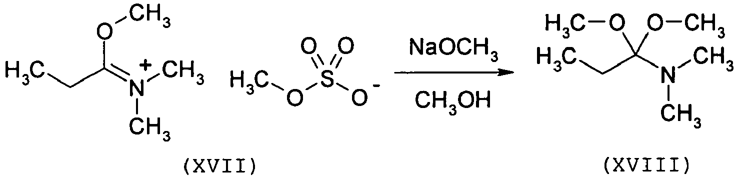

Sodium (3.85 g, 167 mmol) was dissolved in methanol (63.5 g) and cooled to -10 °C. Trimethylpropionic imidinium methylsulfate (XVII; 35 g, 154 mmol) was added drop wise such that the temperature did not rise above -5 °C, whereby a white suspension was formed. The mixture was stirred and warmed to 22 °C.

Et₂O (45 g) was added and the formed salts filtered off. The residue was washed with Et₂O (45 g). The filtrate was concentrated, whereupon more salts precipitated. The mixture was filtered again and washed with Et₂O (45 g). The filtrate was concentrated and purified by distillation to afford 9.5 g (main fraction) of 1,1-Dimethoxy-*N*,*N-*dimethyl-propan-1-amine (XVIII) in 82% purity according to GC, amounting to 34% yield.

### Example 4: (2RS*,3RS*)-3-(3-Methoxyphenyl)-N,N,2-tri-methylpent-4-enamide ±-(XIX) (Option 1)

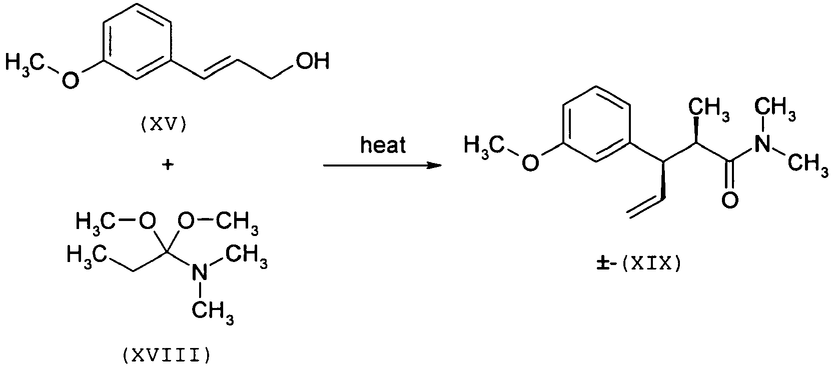

(*E*)-3-(3-methoxyphenyl)prop-2-en-1-ol (XV; 2.5 g, 15 mmol) and 1,1-dimethoxy-*N*,*N*-dimethylpropan-1-amine (XVIII; 4.5 g, 25 mmol) in xylene (43 g) were heated to reflux (120 °C) and stirred for 16 h.

Then the mixture was cooled to 22 °C and water (30 g) was added and stirred for 1 h. The phases were separated and the organic phase was concentrated in vacuo to afford 3.5 g of (2RS*,3RS*)-3-(3-methoxyphenyl)-*N*,*N*,2-trimethylpent-4-enamide ±-(XIX) as a yellow oil in 93% yield and a diastereomeric ratio of 75:25.

### Example 5 : (2RS*,3RS*)-3-(3-Methoxyphenyl)-N,N,2-trimethylpent-4-enamide ±-(XIX) (Option 2)

According to an alternative protocol, the Eschenmoser-Claisen rearrangement is performed using 1-dimethylamino-1-methoxy-prop-l-ene (XX) as the second reagent. 1-dimethylamino-1-methoxy-prop-1-ene (XX) can be prepared according to the protocol published by Bredereck et al. in Chemische Berichte, 1964, vol. 97, p. 3081-3087.

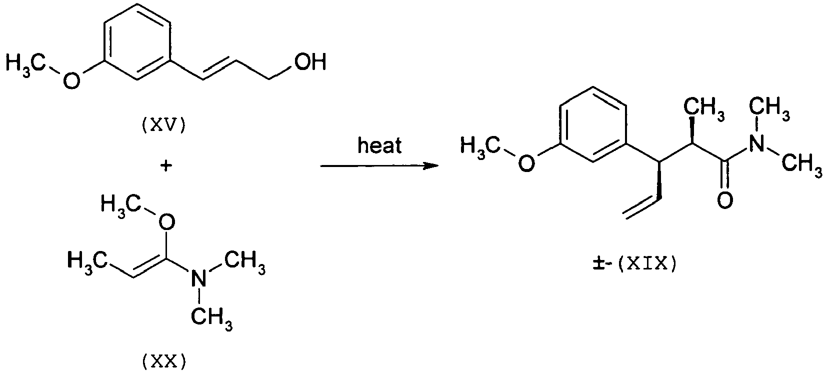

(E)-3-(3-methoxyphenyl)prop-2-en-1-ol (XV; 2.5 g, 15 mmol) and 1-dimethylamino-1-methoxy-prop-1-ene (XX; 1.9 g, 16 mmol) in xylene (20 g) were heated to reflux (120 °C) and stirred for 16 h.

Then the mixture was cooled to 22 °C, water (30 g) added and stirred for 1 h. The phases were separated and the organic phase was concentrated in vacuo to afford 3.5 g of (*2RS**,*3RS**)-3-(3-methoxyphenyl)-*N*,*N*,2-trimethyl-pent-4-enamide ±-(XIX) as a yellow oil in 93% yield and a diastereomeric ratio of 92:8.

### Example 6: (2RS*,3RS*)-3-(3-Methoxyphenyl)-N,N,2-trimethylpent-4-en-1-amine ±-(XXI)

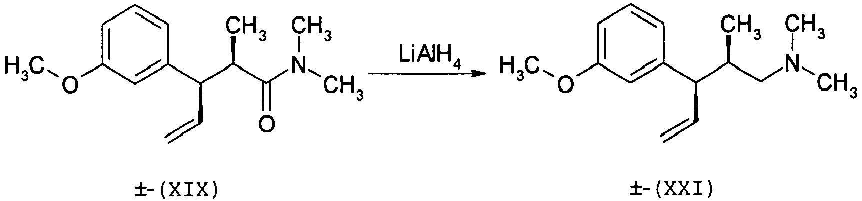

A mixture of (*2RS**,*3RS**)-3-(3-methoxyphenyl)-*N*,*N*,2-trimethylpent-4-enamide (±-(XIX); 106 g, 429 mmol) in THF (500 ml) was added within 40 minutes at 0 °C to a mixture of LiAlH₄ (16.3 g, 429 mmol) in THF (220 ml) The resulting mixture was then stirred for 16 h at 48 °C and then cooled to -5 °C.

The reaction was stopped by the addition of water (120 ml), and then 15% aq. NaOH-solution (120 ml) was added at 5 - 10 °C, followed by addition of water (360 g). After decanting of the liquid, the residual solid was treated with AcOEt (300 ml) and the liquid again decanted. The combined liquids were concentrated in vacuo.

The residue was treated with AcOEt (300 ml), water (350 ml), and 32% aq. HCl-solution (50 ml). The phases were separated and the aqueous phase extracted again twice with AcOEt (200 ml). The water phase was then treated with 30% aq. NaOH-solution and extracted twice with AcOEt (300 ml and 200 ml). The organic phase was then washed with water (200 ml) and dried over Na₂SO₄, filtered and concentrated in vacuo to afford 80.0 g of (*2RS*,3RS**)*-3-*(3-methoxyphenyl)-*N*,*N*,2-tri-methylpent-4-en-1-amine ±-(XXI) as a brown oil in 80% yield.

### Example 7: Chiral Resolution of (2RS*,3RS*)-3-(3-Methoxyphenyl)-N,N,2-tri-methylpent-4-en-1-amine ±-(XXI)

The separation of the two enantiomers of (*2RS**,*3RS**)-3-(3-methoxyphenyl)-*N*,*N*,2-tri-methylpent-4-en-1-amine ±-(XXI) was brought about by chiral resolution using L-di-p-toluoyltartaric acid (XXII):

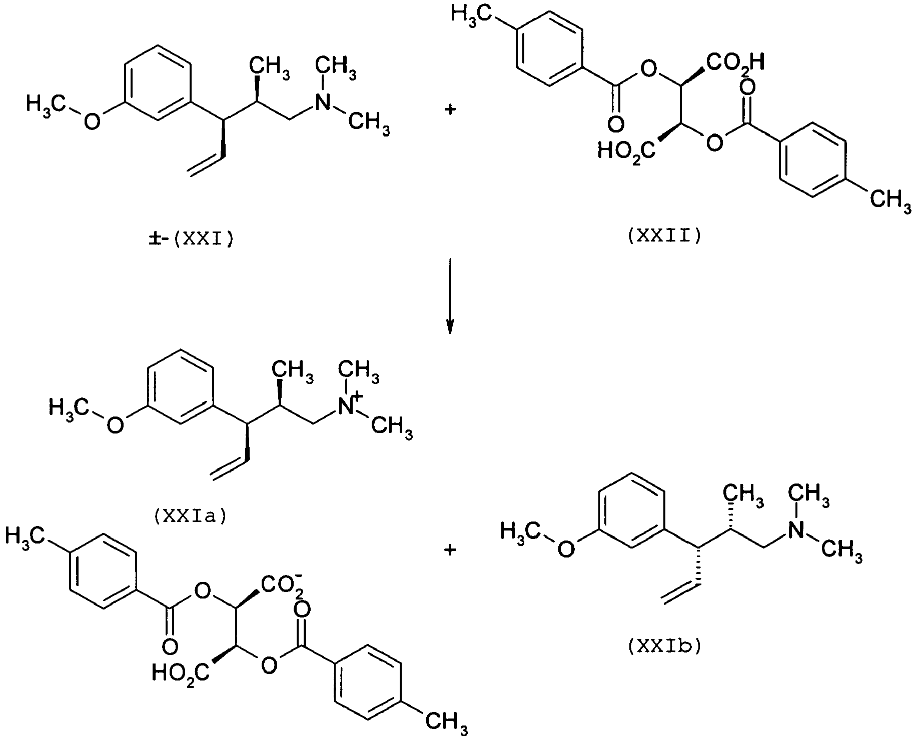

(*2RS**,*3RS**)-3-(3-methoxyphenyl)-*N*,*N*,2-trimethylpent-4-en-1-amine (±-(XXI); 5 g, 21.4 mmol) was dissolved in a mixture of water (50 g) and acetone (50 g) at 55 °C. Then L-di-*p*-toluoyltartaric acid (XXII; 8 g, 20.7 mmol) was added to the mixture. The mixture was stirred until a solution was obtained at 58 °C, and then cooled to 46 °C. Crystallization was induced by self-seeding, and then the mixture was cooled to 28 °C and filtered.

The residue was washed with acetone and dried at 50 °C in vacuo to afford 6.6 g of (2R,3R)-3-(3-methoxyphenyl)-*N*,*N*,2-trimethylpent-4-en-1-ammonium L-di-p-toluoyltartrate as white crystals in 35% yield.

### Example 8: Preparation of (2R,3R)-3-(3-Methoxyphenyl)-N,N,2-trimethylpentan-1-amine (XXIIIa)

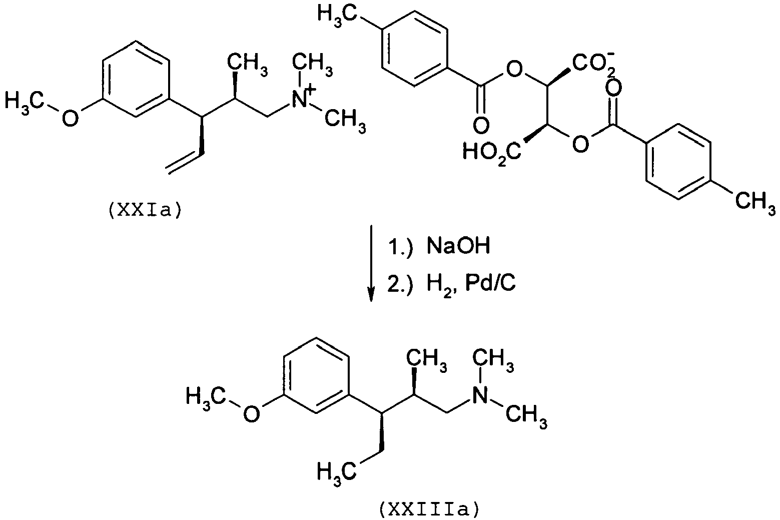

(*2R*,*3R*)-3-(3-methoxyphenyl)-*N*,*N*,2-trimethylpent-4-en-1-ammonium L-di-p-toluoyltartrate (6.1 g, 9.8 mmol) was mixed with water (30 ml), 30% aq. NaOH-solution (5 ml), and CH₂Cl₂ (30 ml) and extracted. The organic phase was dried over Na₂SO₄ and concentrated in vacuo to give 2.3 g of (2R,3R)-3-(3-methoxyphenyl)-N,N,2-tri-methylpent-4-en-1-amine (XXIa).

The latter was hydrogenated in ethanol (40 g) using 5% Pd/C catalyst (0.4 g) for 22 h under 5 bars H₂ at 24 °C. The catalyst was then filtered off and the resulting solution concentrated in vacuo to afford 2.3 g of (2R,3R)-3-(3-methoxyphenyl)-*N*,*N*,2-trimethylpentan-1-amine (XXIIIa) as a colorless oil in 99% yield.

### Example 9: Preparation of 3-[(1R,2R)-3-(Dimethylamino)-1-ethyl-2-methylpropyl]phenol (IXa) (tapentadol)

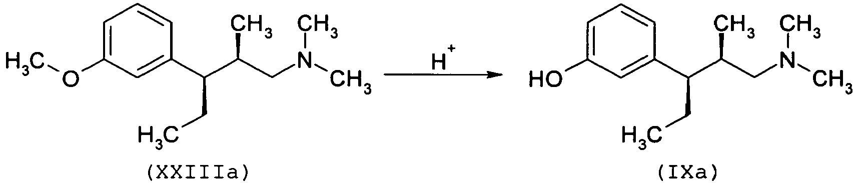

(*2R*,*3R*)-3-(3-methoxyphenyl)-N,N,2-trimethylpentan-1-amine (XXIIIa; 1.7 g, 7.2 mmol) and DL-methionine (1.3 g, 8.8 mmol) were stirred in methanesulfonic acid (7 ml) at 80 °C for 16 h. After cooling to 20 °C, water (4.5 g) was added (exothermic) and the pH adjusted to 11 by addition of 30% aq. NaOH-solution.

The mixture was extracted with AcOEt (30 g) and the organic phase washed with water (20 g) and concentrated in vacuo to give 1.3 g of 3-[(*1R*,*2R*)-3-(dimethylamino)-1-ethyl-2-methylpropyl]phenol (IXa) (tapentadol) as a yellow oily product in 81% yield.

### Example 10: Use of an Ireland-Claisen rearrangement

As an alternative to the above described Eschenmoser-Claisen rearrangement, the 1-aryl-1-alkyl-2-alkyl-3-dialkylaminopropane compounds may also be prepared via an Ireland-Claisen rearrangement.

In this respect, Godenschwager and Collum have disclosed the following reaction (J. Am. Chem. Soc. 2008, 130, 8726-8732):

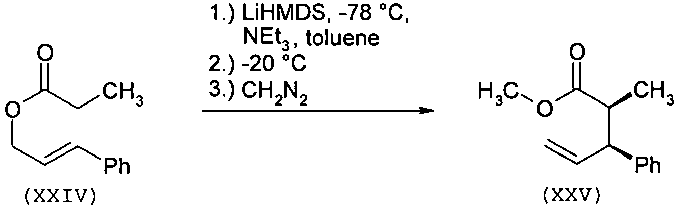

As described in Scheme 2 (see above), the carboxylic acid ester (XXV) can then be converted to the corresponding amide and finally the amine in order to obtain the 1-aryl-1-alkyl-2-alkyl-3-dialkylaminopropane compound.

## Claims

1. Method for the preparation of a 1-aryl-1-alkyl-2-alkyl-3-dialkylaminopropane compound having the general formula (I) 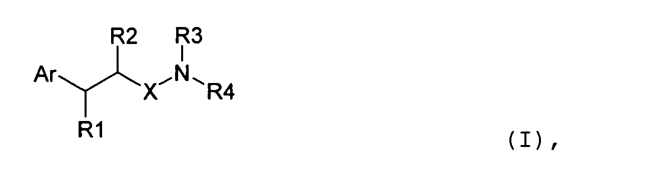 wherein
Ar is a substituted or unsubstituted aryl group, in particular a substituted phenyl group,
R1 is a linear, branched or cyclic C₂ to C₄ alkyl or alkenyl group,
R2 is a linear, branched or cyclic C₁ to C₄ alkyl group,
R3 and R4 each are independently of one another a linear, branched or cyclic C₁ to C₄ alkyl group, hydrogen or an optionally substituted benzyl group or R3 and R4 together form a cyclopentyl, cyclohexyl, tetrahydrofuryl or tetrahydropyryl group, and
X is a methylene or a carbonyl group,
**characterized in that** the method involves an Eschenmoser-Claisen or an Ireland-Claisen rearrangement, preferably an Eschenmoser-Claisen rearrangement.

2. Method according to claim 1, **characterized in that** a
compound of formula (Ia) 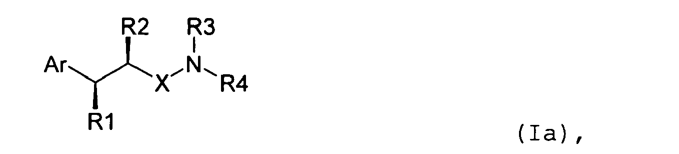 the other enantiomer of (Ia) or a mixture of the two enantiomers is formed,
wherein
Ar, R1, R2, R3, R4, and X are as defined above.

3. Method according to one of the preceding claims, **characterized in that** it involves reacting a compound of formula (II) 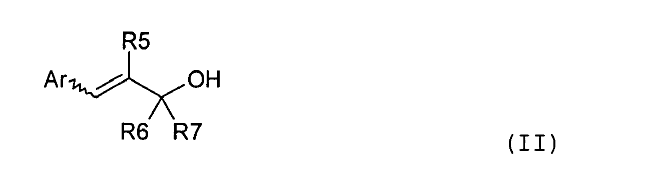 with a compound of formula (III) or (IV) 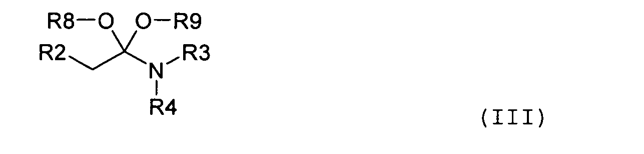 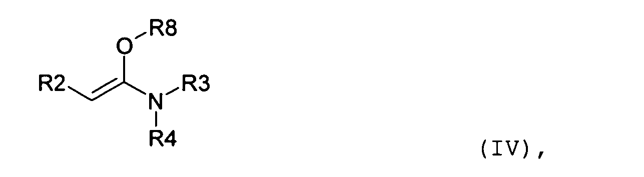 to afford a compound of formula (V) 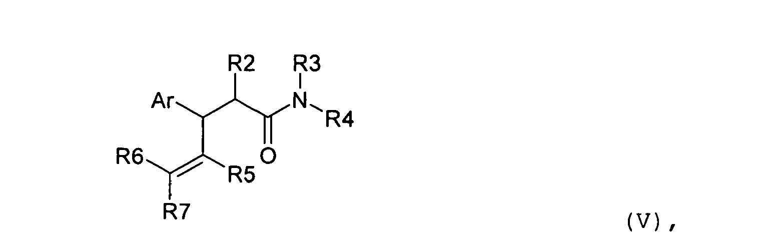 more preferably to afford a compound of formula (Va) 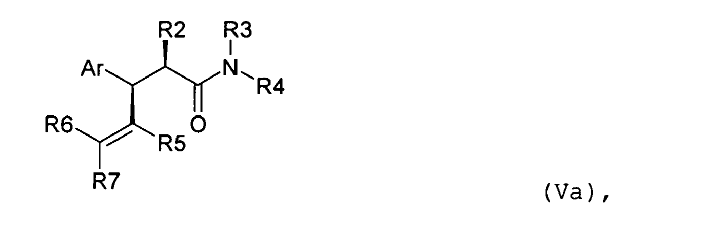 the other enantiomer of (Va) or a mixture of the two enantiomers,
wherein
Ar, R2, R3, and R4 are as defined above,
R5, R6, and R7 are independently of one another selected from the group consisting of hydrogen and methyl, and
R8 and R9 are independently of one another a linear, branched or cyclic C₁ to C₄ alkyl group or optionally a substituted benzyl group.

4. Method according to one of the preceding claims, **characterized in that** Ar is selected from the group consisting of 3-hydroxy-phenyl, 3-methoxyphenyl, 3-benzyloxyphenyl, and O-protected 3-hydroxyphenyl.

5. Method according to one of the preceding claims, **characterized in that** R1 is selected from the group consisting of ethyl, ethenyl, propyl, propenyl, iso-propyl, *iso*-propenyl, butyl, butenyl, *iso*-butyl, and *iso*-butenyl, and is preferably an ethyl or an ethenyl group.

6. Method according to one of the preceding claims, **characterized in that** R2 is selected from the group consisting of methyl, ethyl, propyl, *iso*-propyl, butyl, and *iso*-butyl, and is preferably a methyl group.

7. Method according to one of the preceding claims, **characterized in that** R3 and R4 are independently of one another selected from the group consisting of methyl, ethyl, propyl, iso-propyl, butyl, *iso*-butyl, and optionally substituted benzyl, and are preferably methyl groups.

8. Method according to one of the preceding claims,
**characterized in that** a compound of formula (VI) 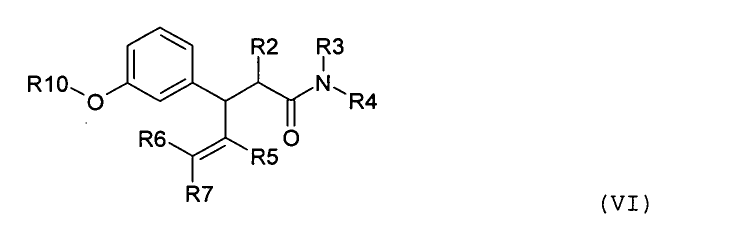 is formed,
more preferably a compound of formula (VIa) 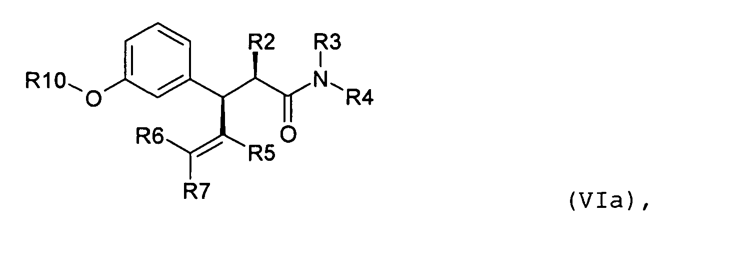 the other enantiomer of (VIa) or a mixture of the two enantiomers,
wherein
R2, R3, R4, R5, R6, and R7 are as defined above, and R10 is selected from the group consisting of hydrogen, methyl, benzyl, and an alcohol protecting group, and
wherein preferably
R2, R3, R4, and R10 are methyl and R5, R6, and R7 are hydrogen.

9. Method according to one of the preceding claims,
**characterized in that** a compound of formula (VII) 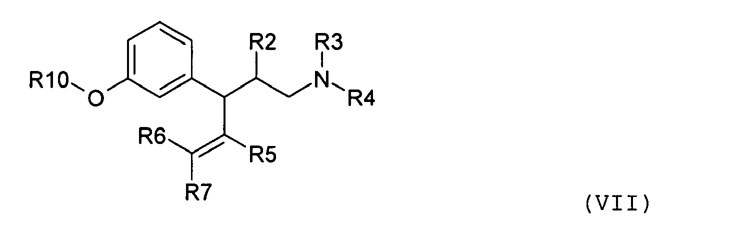 is formed,
more preferably a compound of formula (VIIa) 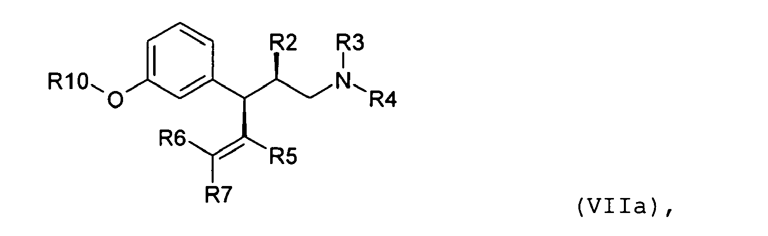 the other enantiomer of (VIIa) or a mixture of the two enantiomers,
wherein
R2, R3, R4, R5, R6, and R7 are as defined above, and R10 is selected from the group consisting of hydrogen, methyl, benzyl, and an alcohol protecting group, and
wherein preferably
R2, R3, R4, and R10 are methyl and R5, R6, and R7 are hydrogen.

10. Method according to one of the preceding claims,
**characterized in that** a compound of formula (VIII) 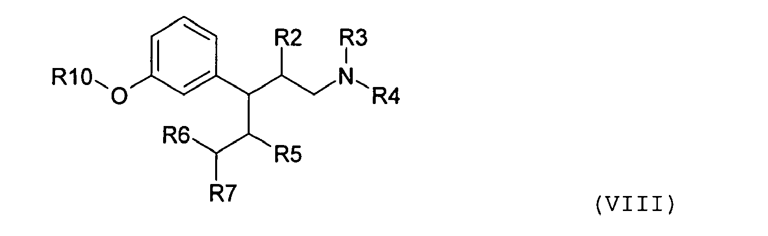 is formed,
more preferably a compound of formula (VIIIa) 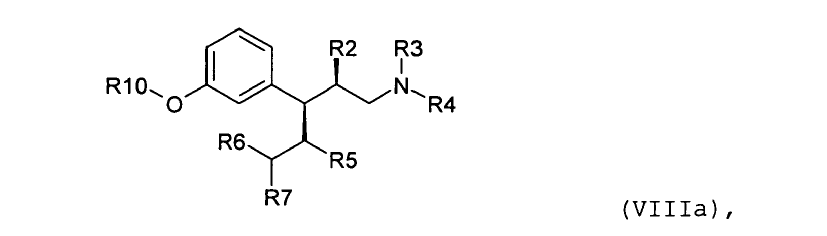 the other enantiomer of (VIIIa) or a mixture of the two enantiomers,
wherein
R2, R3, R4, R5, R6, and R7 are as defined above, and R10 is selected from the group consisting of hydrogen, methyl, benzyl, and an alcohol protecting group, and
wherein preferably
R2, R3, R4, and R10 are methyl and R5, R6, and R7 are hydrogen.

11. Method according to one of the preceding claims,
**characterized in that** the compound of formula (IX) 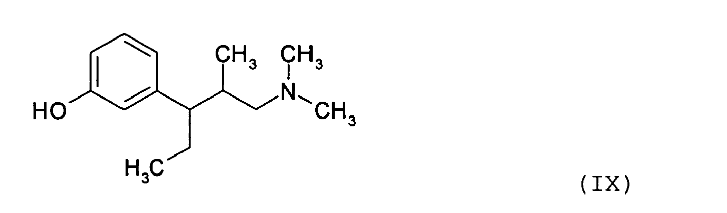 is formed,
more preferably a compound of formula (IXa) 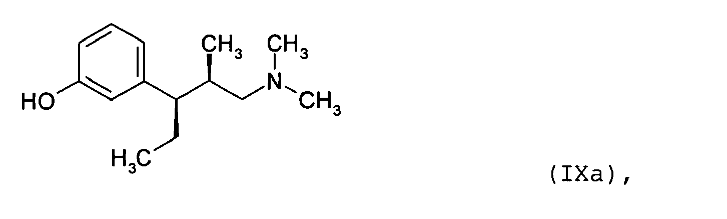 the other enantiomer of (IXa) or a mixture of the two enantiomers,
most preferably only (IXa).

12. Use of an Eschenmoser-Claisen or an Ireland-Claisen rearrangement for the preparation of tapentadol (IXa).

13. A compound of formula (VI) 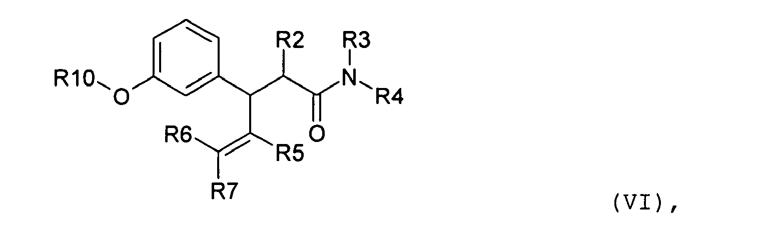 more preferably a compound of formula (VIa) 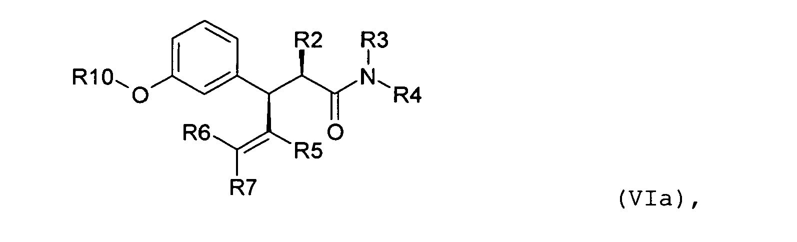 the other enantiomer of (VIa) or a mixture of the two enantiomers,
wherein
R2 is a linear, branched or cyclic C₁ to C₄ alkyl group,
R3 and R4 each are independently of one another a linear, branched or cyclic C₁ to C₄ alkyl group, hydrogen or an optionally substituted benzyl group or R3 and R4 together form a cyclopentyl, cyclohexyl, tetrahydrofuryl or tetrahydropyryl group,
R5, R6, and R7 are independently of one another selected from the group consisting of hydrogen and methyl, and
R10 is selected from the group consisting of hydrogen, methyl, benzyl, and an alcohol protecting group,
wherein preferably
R2, R3, R4, and R10 are methyl and R5, R6, and R7 are hydrogen.

14. A compound of formula (VII) 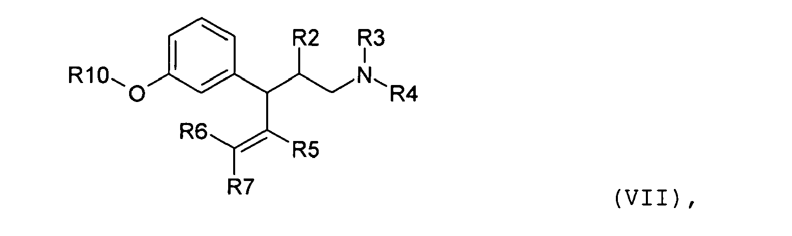 more preferably a compound of formula (VIIa) 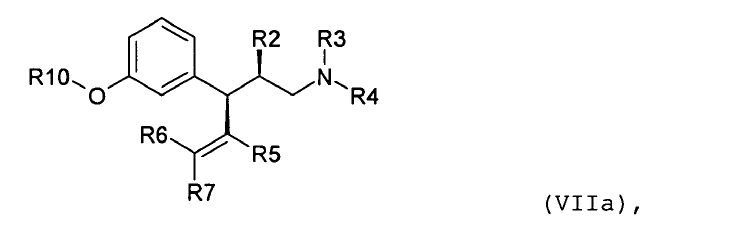 the other enantiomer of (VIIa) or a mixture of the two enantiomers,
wherein
R2 is a linear, branched or cyclic C₁ to C₄ alkyl group,
R3 and R4 each are independently of one another a linear, branched or cyclic C₁ to C₄ alkyl group, hydrogen or an optionally substituted benzyl group or R3 and R4 together form a cyclopentyl, cyclohexyl, tetrahydrofuryl or tetrahydropyryl group,
R5, R6, and R7 are independently of one another selected from the group consisting of hydrogen and methyl, and
R10 is selected from the group consisting of hydrogen, methyl, benzyl, and an alcohol protecting group,
wherein preferably
R2, R3, R4, and R10 are methyl and R5, R6, and R7 are hydrogen.

15. A compound of formula (VIII) 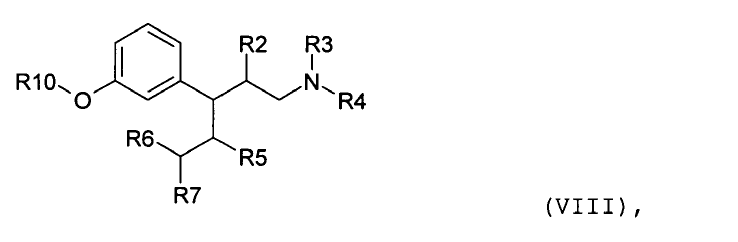 more preferably a compound of formula (VIIIa) 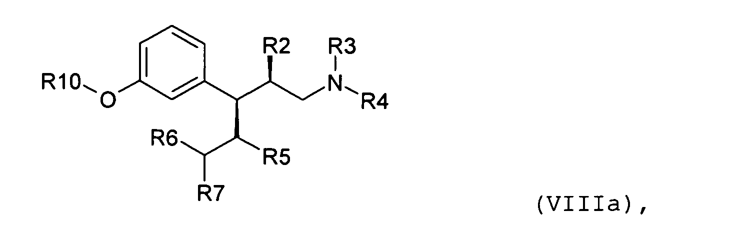 the other enantiomer or a mixture of the two enantiomers,
wherein
R2 is a linear, branched or cyclic C₁ to C₄ alkyl group,
R3 and R4 each are independently of one another a linear, branched or cyclic C₁ to C₄ alkyl group, hydrogen or an optionally substituted benzyl group or R3 and R4 together form a cyclopentyl, cyclohexyl, tetrahydrofuryl or tetrahydropyryl group,
R5, R6, and R7 are independently of one another selected from the group consisting of hydrogen and methyl, and
R10 is selected from the group consisting of hydrogen, methyl, benzyl, and an alcohol protecting group,
wherein preferably
R2, R3, R4, and R10 are methyl and R5, R6, and R7 are hydrogen.
